(19) Europäisches Patentamt
European Patent Office
Office européen des brevets

(11) Publication number: **0 210 753 A2**

(12) **EUROPEAN PATENT APPLICATION**

(21) Application number: 86304966.4

(22) Date of filing: 26.06.86

(51) Int. Cl.⁴: A 61 K 31/415
A 61 K 31/44, C 07 D 213/64
C 07 D 213/65

(30) Priority: 26.06.85 JP 140901/85

(43) Date of publication of application: 04.02.87 Bulletin 87/6

(84) Designated Contracting States: AT BE CH DE FR GB IT LI LU NL SE

(71) Applicant: YAMANOUCHI PHARMACEUTICAL CO. LTD.
No. 5-1 Nihonbashi-Honcho, 2-chome Chuo-ku
Tokyo(JP)

(72) Inventor: Numasaki, Yoso
Nishiageo daiichi dancho 3-23-206
Oaza Koshikiya 845-1 Ageo-shi Saitama(JP)

(72) Inventor: Takahashi, Koichiro
No. 4-4-2-812, Kita kasai
Edogawa-ku Tokyo(JP)

(72) Inventor: Ohata, Isao
No. 47-17, Marugasaki-cho
Omiya-shi Saitama(JP)

(74) Representative: Geering, Keith Edwin et al,
REDDIE & GROSE 16 Theobalds Road
London WC1X 8PL(GB)

(54) **Anti-tumor medicament.**

(57) This disclosure describes compositions of matter useful as growth inhibitors of transplanted tumors in mammals; and discloses the use of such compositions in medicaments for inducing the prevention of, regression and/or palliation of, various types of tumors in mammals (mammary cancer, liver cancer, skin cancer, etc.), said medicaments comprising a compound of the following formula:

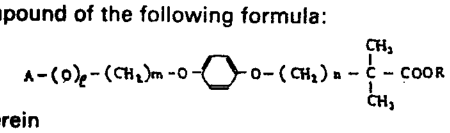

wherein

A represents an imidazolyl group or a pyridyl group,
$\ell$ represents 0 to 1,
m and n represent the same or different,
represents an integer of 1 to 6, and,
R represents a hydrogen atom or a lower alkyl group
or a salt thereof:

The above formula compounds have low toxicity, and it is expected to apply various types of administration thereof such as oral administration and parenteral administration. In particular, it is expected that the compounds are useful as new type of medical (anti-cancer) compounds which can be administered orally.

EP 0 210 753 A2

ANTI-TUMOR MEDICAMENT

This invention relates to a medicament for preventing or treating (inducing the regression and/or palliation of) tumors in mammals (mammary cancer, liver cancer, skin cancer, etc.), and for reducing or preventing metastasis of such tumors, said medicament comprising an effective amount of compound formula (I)

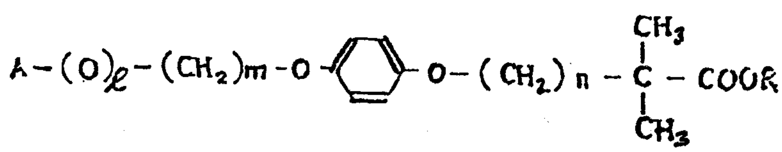 (I)

wherein A represents an imidazolyl group or a pyridyl group,

ℓ represents 0 or 1,

m and n represent the same or different integers of 1 to 6, and

R represents a hydrogen atom or a lower alkyl group

or a salt thereof; the medicament is also effective for prevention or inhibition of metastasis of the various tumors.

Among the compounds which fall under the above formula (I) some ω-(p-substituted phenoxy)-2,2-dimethylalkanoic acid esters are described in Published Japanese Application 6667-85 and EP-A-130077,

together with physical data thereon and mention of the medical utility of these compounds for inhibiting platelet agglutination.

In the definition of the groups appearing in the formulae in the specification, the term "lower" refers to a straight or branched carbon chain having 1 to 5 carbon atoms, preferably 1 to 3 carbon atoms. Accordingly, specific examples of lower alkyl groups include a methyl group, an ethyl group, a propyl group, an isopropyl group, a butyl group, an isobutyl group, a sec-butyl group, a tert-butyl group, a pentyl (or amyl) group, an isopentyl group, a neopentyl group, a tert-pentyl group, etc.

The compounds represented by general formula (I) above form salts, which the present invention encompasses.

In particular, preferred examples of the salts include salts with inorganic acids such as hydrochloric acid, hydrobromic acid, phosphoric acid, etc.; salts with organic acids such as formic acid, acetic acid, lactic acid, oxalic acid, succinic acid, fumaric acid, benzoic acid, benzenesulfonic acid, etc. and quaternary ammonium salts with alkyl halides such as methyl iodide, etc.

The compounds of the formula (I) and salts thereof can be prepared by the methods described in the above-mentioned published JP and EP specifications which disclose some of the formula (I) compounds with Examples for their production.

As a result of investigation of the pharmacological activity of ω-(p-substituted phenoxy)-2,2-dimethylalkanoic acids and esters and salts thereof, we have found that the compounds of formula (I) possess anti-tumor activity and extremely low toxicity.

In animal tests using mice, compounds of formula (I) showed

excellent inhibition effects on various tumors such as Ehrlich solid tumor, MM-46 solid tumor, Meth A solid tumor, Ehrlich ascites tumor, etc., and their toxicity is extremely low. Therefore, the compounds of formula (I) can be used as safe and potent anti-tumor agents or as agents for inhibiting metastasis of tumors.

Representative examples of formula (I) compounds include :

Methyl 5-[p[3-(1-imidazolyl)propoxy]phenoxy]-2,-2-dimethylpentanoate (Compound A)

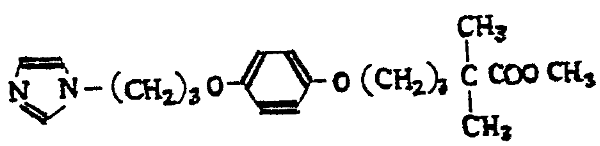

Ethyl 6-[p-[5-(1-imidazolyl)pentyloxy]phenoxy]-2,2-dimethylhexanoate (Compound B)

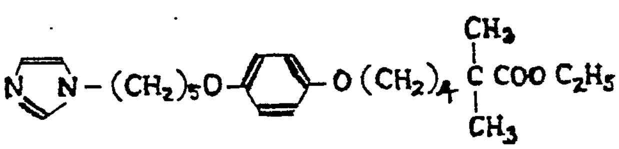

Ethyl 7-[p-[5-(1-imidazolyl)pentyloxy]phenoxy]-2,2-dimethylheptanoate (Compound C) [hydrochloride]

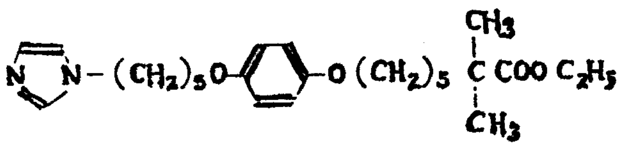

Ethyl 7-[p-[3-(1-imidazolyl)propoxy]phenoxy]-2,2-dimethyl-heptanoate (Compound D)

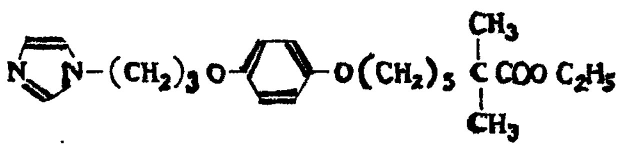

Methyl 7-[p-[3-(1-imidazolyl)propoxy]phenoxy]-2,2-dimethylheptanoate (Compound E)

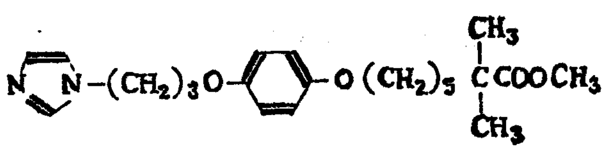

5-[p-[3-(1-Imidazolyl)propoxy]phenoxy]-2,2-dimethylheptanoic acid (Compound F)

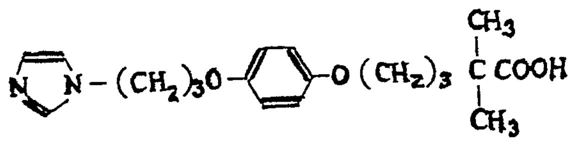

6-[p-[5-(1-Imidazolyl)pentyloxy]phenoxy]-2,2-dimethylhexanoic acid (Compound G)

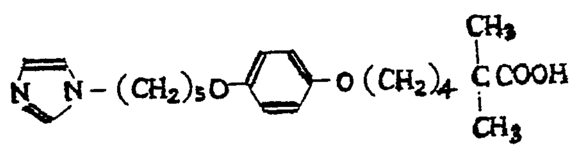

Methyl 7-[p-[3-(3-pyridyloxy)propoxy]phenoxy]-2,2-dimethylheptanoate (Compound H)

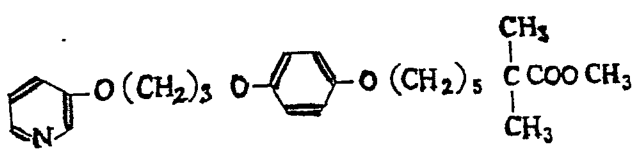

Methyl 2,2-dimethyl-5-[p-[3-(3-pyridyloxy)propoxy]-phenoxy]pentanoate (Compound I)

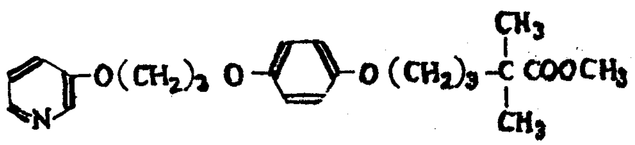

Ethyl 6-[p-[5-(2-pyridyloxy)pentyloxy]phenoxy]-2,2-dimethylhexanoate (Compound J)

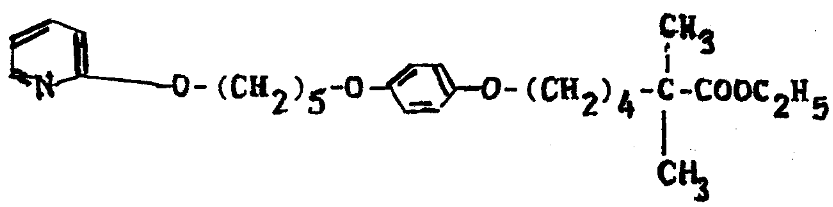

Some formula (I) compounds (e.g. F, G and J) are novel over those disclosed in EP-A-130077.

The pharmacological activity, toxicity, etc. of formula (I) compounds are shown below, together with test methods used.

Anti-tumor test:

(1) Anti-tumor test against Ehrlich solid tumor.

Ehrlich tumor cells, 2 x $10^6$, on Day 7 after intraperitoneal transplantation to ddY/SLC mice, were subcutaneously transplanted in the left abdomen of ddY/SLC mice (5 week age, male) - 10 to a group. The active component was intraperitoneally or orally administered in a definite dose 24 hours after the transplantation, according to the scheduled shown in Table 1 (A),(B) or (C). The effectiveness in

inhibiting tumor growth was assessed by measuring the long diameter and short diameter of each tumor 14, 20 and 21 days after the transplantation, calculating the tumor size ($mm^2$), and determining a tumor growth inhibition rate of the group administered with the active component to the control group according to the following equation :

$$\text{Growth inhibition rate (\%)} = (C-T)/C \times 100$$

T : mean tumor size ($mm^2$) in the active component-administered group

C : mean tumor size ($mm^2$) in the control group

Table 1 (A)

| Test Compound | Dose (mg/kg) | Route for Administration and Days Given | Day 14 Size of Tumor ($mm^2$) | Day 14 Growth Inhibition Rate (%) | Day 21 Size of Tumor ($mm^2$) | Day 21 Growth Inhibition Rate (%) |
|---|---|---|---|---|---|---|
| Compound B | 60 | intraperitoneal administration (1,2,3,4,7,8,10 & 13 Days) | 107.3 ± 54.6 | 25.7 | 323.3 ± 163.4 | 37.0 |
| | 100 | intraperitoneal administration (1,3,7, 10, 13 & 14 Days) | 70.1 ± 33.8 | 51.4** | 315.3 ± 235.3 | 38.5 |
| | 200 | p.o. (1,2,3,4,7,8,10 & 13 Days) | 48.7 ± 44.3 | 66.3** | 109.2 ± 133.5 | 78.7*** |
| 5-Fluorouracil | 25 | intraperitoneal administration (1,2,3,4.& 7 Days) | 86.7 ± 35.1 | 40.0 | 217.8 ± 126.2 | 57.5** |
| Control | | | 144.4 ± 79.0 | | 512.8 ± 271.3 | |

** $p<0.01$, *** $p<0.001$

Table 1 (B)

| Test Compound | Dose (mg/kg) | Route for Administration and Days Given | Day 14 Size of Tumor ($mm^2$) | Day 14 Growth Inhibition Rate (%) | Day 21 Size of Tumor ($mm^2$) | Day 21 Growth Inhibition Rate (%) |
|---|---|---|---|---|---|---|
| Compound C | 25 | intraperitoneal administration (1,2,3,5,6,7 & 9 Days) | 191.1 ± 88.8 | 18.4 | 363.1 ± 188.8 | 31.9* |
| | 50 | intraperitoneal administration (") | 135.0 ± 66.9 | 42.4** | 207.9 ± 139.7 | 61.0*** |
| | 100 | intraperitoneal administration (") | 107.3 ± 46.7 | 54.2*** | 190.7 ± 69.1 | 64.2*** |
| Compound I | 25 | intraperitoneal administration (") | 164.9 ± 96.2 | 29.6* | 336.6 ± 160.0 | 36.9* |
| | 50 | intraperitoneal administration (") | 172.8 ± 82.9 | 26.2 | 369.1 ± 203.5 | 30.7* |
| | 100 | intraperitoneal administration (") | 148.7 ± 66.8 | 36.5 | 290.2 ± 115.5 | 45.6** |
| 5-Fluorouracil | 25 | intraperitoneal administration (") (1,2,3,5 & 6 Days) | 133.7 ± 61.1 | 42.9** | 306.8 ± 166.8 | 42.4** |
| | 1.0 | intraperitoneal administration (") | 75.6 ± 31.5 | 67.7*** | 106.8 ± 62.2 | 80.0*** |
| Control | | | 234.2 ± 82.1 | | 323.3 ± 141.5 | |

* $p < 0.05$, ** $p < 0.01$ *** $p < 0.001$

Table 1 ( C )

| Test compound | Dose (mg/kg) | Route for Administraion and Days Given | Day ; 14 Size of Tumor ($mm^2$) | Day ; 14 Growth Inhibition Rate (%) | Day 21 Size of Tumor ($mm^2$) | Day 21 Growth Inhibtion Rate (%) |
|---|---|---|---|---|---|---|
| Compound J | 50 | intraperitoneal administration (1,2,3,4,6,7,8 9 Days) | $201.7 \pm 48.2$ | 20.9 | $344.7 \pm 108.4$ | 19.2 |
| | 100 | the same as above | $166.5 \pm 65.3$ | 34.7 | $263.5 \pm 97.1$ | 38.2 |
| 5-Fluorouracil | 12.5 | the same as above | $142.6 \pm 36.9$ | 44.1 | $280.6 \pm 68.9$ | 34.2 |
| | 25 | the same as above | $116.3 \pm 44.0$ | 54.4 | $252.3 \pm 100.3$ | 40.8 |
| Control | | | $255.1 \pm 40.9$ | | $426.5 \pm 89.3$ | |

0210753

(2) Test against mouse mammalian MM-46 solid tumor.

MM-46 Tumor cells, 1 x $10^6$, on day 7 after intraperitonal transplantation to $C_3H$/He/SLC mice, were subcutaneously transplanted to the left abdomen of $C_3H$/He/SLC mice (5 week age, male) - 8 mice to a group (21 mice for the control group). The active component was orally administered in a definite dose 24 hours after the transplantation according to the schedule shown in Table 2. The tumor growth inhibition was assessed by measuring the long diameter and short diameter of each tumor on days 14 and 21 after the transplantation, calculating the tumor size ($mm^2$), and determining the tumor growth inhibition rate of the active component-administered group to the control group as before.

Table 2

| Test Compound | Dose (mg/kg) | Route for Administration and Days Given | Day 14 Size of Tumor ($mm^2$) | Day 14 Growth Inhibition Rate (%) | Day 21 Size of Tumor ($mm^2$) | Day 21 Growth Inhibition Rate (%) |
|---|---|---|---|---|---|---|
| Compound B | 100 | p.o. (1-9 Days) | 150.1±52.1 | 40.4*** | 149.4± 84.8 | 55.8** |
| | 200 | 〃 | 147.9±56.2 | 41.3*** | 147.5± 65.3 | 53.4** |
| | 400 | 〃 | 131.5±55.2 | 47.8*** | 157.4±104.0 | 53.4** |
| Tetrahydrofuryl-5-fluorouracil | 50 | 〃 | 216.0±55.1 | 14.2 | 247.0±101.8 | 26.9 |
| | 100 | 〃 | 214.8±55.6 | 14.7 | 250.1± 92.9 | 26.0 |
| Control | | | 251.9±71.2 | | 338.0±151.6 | |

* $p<0.05$, ** $p<0.01$, *** $p<0.001$

(3) Anti-tumor test against methyl cholanathrene-induced Meth A solid tumor.

Meth A tumor cells, $1.5 \times 10^6$, on day 7 after intraperitoneal transplantation to Balb/C/SLC mice, were subcutaneously transplanted to the left abdomen of Balb/C/SLC mice (5 week age, male) - 8 mice to a group (16 mice for the control group). The active component was orally administered in a definite dose 24 hours after the transplantation according to the schedule shown in Table 3. The tumor growth inhibition was assessed by measuring the long diameter and short diameter of each tumor on days 15 and 21 after the transplantation, calculating the tumor size ($mm^2$), and determining the tumor growth inhibition rate of the active component-administered group to the control group as before.

Table 3

| Test Compound | Dose (mg/kg) | Route for Administration and Days Given | Day 15 Size of Tumor ($mm^2$) | Day 15 Growth Inhibition Rate (%) | Day 21 Size of Tumor ($mm^2$) | Day 21 Growth Inhibition Rate (%) |
|---|---|---|---|---|---|---|
| Compound B | 100 | p.o. (1-8 Days) | 163.0±41.9 | 27.5 | 387.9±101.8 | 18.8 |
| | 200 | 〃 | 124.5±60.2 | 46.4** | 290.8±100.9 | 39.2* |
| | 400 | 〃 | 104.4±56.9 | 53.6** | 279.3±152.1 | 41.6** |
| Tetrahydrofuryl-5-fluorouracil | 50 | 〃 | 123.4±69.9 | 45.1* | 235.8±124.4 | 50.7** |
| | 100 | 〃 | 93.1±50.3 | 58.6** | 264.4±110.6 | 44.7** |
| Control | | | 224.6±89.8 | | 477.7±189.8 | |

* $p<0.05$, ** $p<0.01$

(4) Anti-tumor effect against Ehrlich ascites tumor.

Ehlrich tumor cells, 1 x $10^6$, on day 7 after intraperitoneal transplantation to ddY/SLC mice, intraperitoneally transplanted to ddY/SLC mice (5 week age, male) - 10 mice to a group. The active component was intraperitoneally administered in a definite dose 24 hours after the transplantation according to the schedule shown in Table 4. The tumor growth inhibition was evaluated as the percentage of the median survival day of the administered group versus that of the control group, according to the following equation.

$$T/C\ \% = T/C \times 100$$

T: median survival day of the administered group

C: median survival day of the control group.

Table 4

| Test Compound | Dose (mg/kg) | Route for Administration and Days Given | Intermediate Survival Day | T/C(%) | Alive Mice (40 days) |
|---|---|---|---|---|---|
| Compound B | 15 | intraperitoneal administration (1-9 Days) | 16.0 | 106 | 0/8 |
| | 30 | " | 22.5 | 150.0 | 1/8 |
| | 60 | " | 21.5 | 143.3 | 2/8 |
| | 100 | intraperitoneal administration (1,3,6,8 & 10 Days) | 26.5 | 176.7 | 2/8 |
| 5-Fluorouracil | 25 | intraperitoneal administration (1-9 Days) | 24.5 | 163.3 | 0/8 |
| Control | | | 15.0 | 100 | 0/10 |

(5) Anti-tumor test against mice mammary cancer MM-46 ascites tumor.

MM-46 tumor cells, $1 \times 10^6$, on day 7 after intraperitoneal transplantation to $C_3H$/He/CRJ mice were intraperitoneally transplanted to $C_3H$/He/CRJ mice (5 week age, male) - 10 mice to a group. The active component was intraperitoneally administered in a definite dose 24 hours after the transplantation according to the schedule shown in Table 5. The tumor growth inhibition was evaluated as the percentage of the median survival time of the administered group versus that of the control group, according to the following equation.

$$T/C\ \% = T/C \times 100$$

T: median survival day of the administered group

C: median survival day of the control group.

**Table 5**

| Test compound | Dose (mg/kg) | Route of Administration and Days Given | Median Survival Day | T/C (%) | Survivors (90 days) |
|---|---|---|---|---|---|
| Compound B | 6.25 | intraperitoneal administration (1,2,3,4 and 6 Days) | 7.0 | 93.3 | 0/10 |
| | 12.5 | | 7.0 | 93.3 | 0/10 |
| | 25 | | 8.5 | 113.3 | 0/10 |
| | 50 | | 34.0 | 453.3 | 0/10 |
| | 100 | | 49.0 | 653.3 | 1/10 |
| | 200 | | 18.5 | 246.7 | 0/10 |
| 5-Fuorouracil | 6.25 | | 16.0 | 213.3 | 0/10 |
| | 12.5 | | 17.0 | 226.7 | 0/10 |
| | 25 | | 20.0 | 266.7 | 0/10 |
| Control | | | 7.5 | | 0/10 |

It is known that the value of an animal tumor screen depends on its ability to select new drugs with efficacy against human tumors. It is also known that some rodent tumors (for example, L1210, B-16, etc.) would have predicted the clinical activity of most of the established anticancer drugs.

The active ingredients of the present invention inhibit transplanted mammalian tumor growth and induce regression and/or palliation of various cancers (for example, skin cancer, mammary cancer, etc.) [solid tumors, ascites tumors, etc.] in mammals when administered orally or intraperitoneally in amounts ranging from about 12.5 mg to about 400 mg per kilogram of body weight per day.

(6) Inhibition of tumor metastasis:

(a) Inhibition of spontaneous metastasis (to the lymph node) of liver cancer (MH-134) cells in mice MH-134 tumor cells, $1 \times 10^6$, on day 7 after intraperitoneal transplantation to $C_3H$/He/SLC mice, were transplanted to the right leg. On the 11th day after the transplantation, the right leg portion wherein the cancer cells were transplanted was cut off. The test compounds were administered orally in a definite dose 24 hours after the transplantation. In order to confirm metastasis-inhibiting effect, on the 20th day after transplantation the mice were anatomised to observe the metastasis to the lymph node in the inguinal portion.

Metastasis-inhibition rate (%)

$$= 1 - \frac{\text{metastasis occurrence rate (\%) of the administered group}}{\text{metastasis occurrence rate (\%) of the control group}}$$

Further, the weight of the lymph node metastasis tumor in the inguinal portion of anatomised mice was measured.

Growth inhibition rate (%) of metastasis tumor (%)

$$= 1 - \frac{\text{mean weight of the tumor of the administered group}}{\text{mean weight of the tumor of the control group}}$$

Table 6(a)

| Test compound | Dose (mg/kg) | Route for administration and days given | Metastasis-preventing rate (%) | Growth inhibition of the metastasis tumor |
|---|---|---|---|---|
| Compound B | 100 | oral administration (1,3,5,7,9,11,13 and 15 days) | 8.5 | 33.8 |
| | 200 | " | 41.2 | 79.4 |
| | 400 | " | 70.9 | 80.4 |
| 5-Florouracil | 50 | " | 17.7 | 48.7 |
| | 100 | " | 41.2 | 74.2 |

(b) Inhibition of experimental metastasis (to the lung) of melanoma BL-6 in mice.

BL-6 cancer cells, $5 \times 10^4$, were inoculated into the tail veins of male $BDF_1$/SLC mice (6 week age) - 6 mice to a group (13 mice for the control group). The test compounds were administered orally in a definite dose 24 hours after the inoculation. In order to observe the metastasis-inhibitory effect, on the 22nd day after the inoculation, the mice were anatomised to observe the metastasis to the lungs. The degree of metastasis was evaluated by the number of pulmonary nodules.

Metastasis (to the lung) inhibition rate (%)

$$= \frac{C - T}{C} \times 100$$

T: Median number of pulmonary nodules of the administered group

C: Median number of pulmonary nodules of the control group.

Table 6(b)

| Test compound | Dose (mg/kg) | Route for administration and days given | number of pulmonary nodules range | medium value | Pulmonary metastasis-preventing rate (%) |
|---|---|---|---|---|---|
| Compound B | 200 | Oral administration (1,2,3,5,6,7,8,9 and 10 days) | 1-20 | 10.5 | 50.0 |
| | 400 | " | 1-14 | 6.5 | 69.0 |
| Futoraful | 50 | " | 1-49 | 13.5 | 35.7 |
| | 100 | " | 1-16 | 6.5 | 69.0 |
| Control | | | 3-87 | 21.0 | 0 |

From the above results, it is apparent that the compounds of this invention inhibit or prevent metastasis of the various cancers. Thus, the compounds of the formula (I) are expected to be useful for treatment of cancer metastasis.

(7) Effectiveness against mouse tumor cells and human tumor cells (in vitro cytotoxicity test).

(a) against mouse tumor cells.

A suspension of cells in culture medium was transferred into wells in a microplate in an amount of 1 x $10^5$ cells/well. Test sample diluted with a culture medium was added to the suspension in each well, and the mixture was incubated on the medium at 37°C for 3 days in an atmosphere of air containing 5% carbon dioxide. Thereafter, the incubated cells were recovered from the microplate and the number of live cells was counted by the dye exclusion test using a 0.5% solution of trypan blue and by using a hemocytometer. The cytotoxicity effects against various mice tumor cells were evaluated by the mean dose producing 50 %-killing of tumor cells ($IC_{50}$). The cytotoxicity data for the test compound and that of 5-fluorouracil are given in Table 7(a).

Table 7(a)

| | $IC_{50}$ (µg/ml) | |
|---|---|---|
| | Test Compound (Compound B) | 5-fluorouracil |
| L-1210 | 1.9 | 0.06 |
| Ehrlich (carcinoma) | 0.5 | 0.05 |
| MM 46 (mammary cancer) | 0.7 | - |
| MH 134 (liver cancer) | 2.2 | 0.009 |
| Meth A(methylcholanthrene-induced cancer) | 0.8 | 0.1 |

(b) against human tumor cells.

A suspension of cells in a culture medium was transfered into wells in a microplate in an amount of $5 \times 10^4$ cells/well. Test sample diluted with a culture medium was added to the suspension in each well, and the mixture was incubated at 37°C for 3 days in an atmosphere of air containing 5% carbon dioxide. After skimming, remaining cells were dyed with crystalviolet for 10 minutes. After washing with water, 1% sodium lauryl sulfate solution was added to each well, and the absorbancy (optical density) at 540 nm was measured. "% inhibition" was calculated from the following equation.

$$\% \text{ inhibition} = (1 - \frac{\text{optical density (administered group)}}{\text{optical density (control group)}}$$

The cytotoxicity effects against various human tumor cells were evaluated by the mean dose producing 50 %-killing of tumor cells ($IC_{50}$).

Table 7 (b)

| | $IC_{50}$ (μg/ml) | |
|---|---|---|
| | Test Compound (Compound B) | 5-fluorouracil |
| KB (nasal cavity & pharynx cancer) | 1.15 | 1.75 |
| Hela (uterine cancer) | 2.05 | - |
| Bowes (melanoma) | 1.00 | 0.61 |
| ACHN (kidney cancer) | 2.30 | 0.10 |

0210753

Toxicity Test:

The active component was orally administered to ddY mice (mean body weight of 35 g, male). No example of death was noted up to a dose of 5,000 mg/kg. Accordingly, the acute toxicity of Compound B is greater than 5,000 mg/kg.

From the foregoing test results, ω-(p-substituted phenoxy)-2,2-dimethylalkanoic acids, esters and salts thereof possess excellent anti-tumor activity but have low toxicity and are therefore effective for use as anti-tumor agents. A suitable clinical dose of the anti-tumor agent of the present invention is daily 500 to 2,000 mg for adults, preferably 800 to 1,200 mg, in the case of oral administration or in suppository form. The dose is given once or in 2 to 4 sub-doses. The dosage is appropriately varied depending on the condition of the patient, combination use with other agents, age, etc.

In the case of oral administration, medical preparations can be in the form of tablets, capsules, granules or syrups. In the case of parenteral administration, suppositories or injections are applicable. As excipients used for preparation of oral administration, there are lactose, starch, sucrose, talc, magnesium stearate, sorbitol, micro

crystalline cellulose, carboxymethyl cellulose, hydroxypropyl cellulose, etc. As bases used for preparation of suppositories, there are polyethylene glycol, lanoline, cocoa butter, Witep Sol (trademark, manufactured by Dynamit Nobel Co.,Ltd.).

An example of preparing capsules is shown below.

Preparation of capsules :

| | |
|---|---|
| Compound B | 200 mg |
| Lactose | 205 mg |
| Crystalline cellulose | 50 mg |
| Hydroxypropyl cellulose | 15 mg |
| Starch | 25 mg |
| Magnesium stearate | 5 mg |
| | 500 mg |

For the composition described above, the main ingredients, lactose and crystalline cellulose are mixed and an aqueous solution of hydroxypropyl cellulose is added thereto. After the mixture is kneaded, grained and dried, magnesium phosphate is added thereto and mixed. The mixture is filled into No.1 gelatin capsules to prepare capsules.

Preparation of Compound H:

After 110 mg of sodium hydride (60% suspension in

mineral oil) was washed with dry benzene, 10 ml of dry dimethylformamide was added thereto and 264 mg of 3-hydroxypyridine was further added to the mixture with stirring. After vigorous foaming was discontinued, the suspension was heated at 80°C for 30 minutes with stirring and then cooled to room temperature. A solution of 870 mg of methyl 7-[p-[(3-bromopropoxy)phenoxy]-2,2-dimethylheptanoate in 10 ml of dry dimethylformamide was added to the suspension followed by stirring at 60°C for 5 hours. Thereafter the solvent was removed from the reaction solution under reduced pressure. The remaining oily substance was dissolved in benzene washed with an aqueous saturated sodium hydrogen carbonate solution, water and a saturated saline solution in this order and then dried over anhydrous sodium sulfate. After drying, the solvent was distilled off under reduced pressure. The remaining oily substance was subjected to silica gel column chromatography and the product was eluted using an eluent chloroform-methanol solution (50:1). The solvent was distilled off from the eluate under reduced pressure to give the desired methyl 7-[p-[3-(3-pyridyloxy)propoxy]phenoxy]-2,2-dimethylheptanoate as an oily substance.

NMR spectra ($CDCl_3$) (internal standard TMS)

δ ( ppm ) ;

1.16 ( 6H, s, 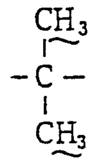

1.2 ~ 1.8 ( 8H, m, 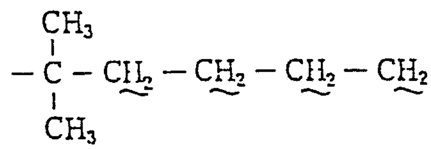 )

2.33 ( 2H, quin, J = 6 Hz, $O-CH_2-CH_2-CH_2-O$ )

3.64 ( 3H, s, 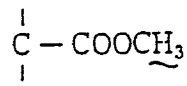 )

3.86 ( 2H, t, J = 6 Hz, 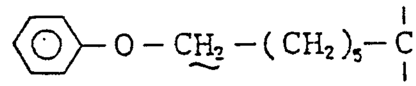 )

4.10 ( 2H, t, J = 6Hz, 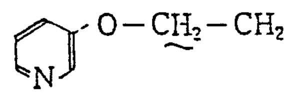

4.18 ( 2H, t, J = 6 Hz, 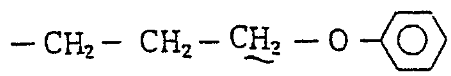 )

6.79 ( 4H, s, 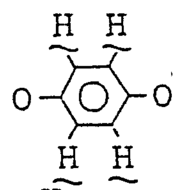 )

7.16 ( 2H, m, 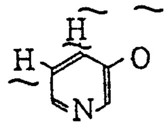 )

8.16 ( 1H, t, J = 2 Hz, 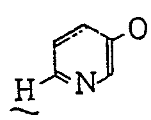 )

8.28 ( 1H, t, J = 2 Hz, 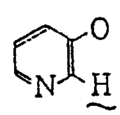 )

Preparation of Compound I:

Using as a starting material methyl 5-[p-[(3-bromopropoxy)phenoxy]-2,2-dimethylpentanoate in place of methyl 7-[p-(3-bromopropoxy)phenoxy]-2,2-dimethylheptanoate in Preparation of Compound H, similar reaction and treatment were conducted to give the desired methyl 2,2-dimethyl-5-[p-[3-(3-pyridyloxy)propoxy]phenoxy]pentanoate as an oily substance.

NMR spectra ($CDCl_3$) (intermal standard TMS)

δ (ppm) ;

1.22 (6H, s, 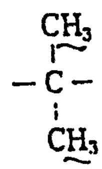 )

1.5 ~ 1.8 (4H, m, 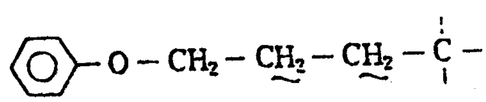

2.2 6 ( 2 H, t, J = 5.8 Hz, 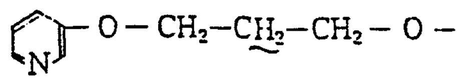 )

3.6 8 ( 3 H, s, $-COOCH_3$ )

3.8 8 ( 2 H, m, 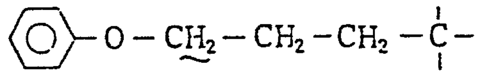 )

4.1 2 ( 2 H, t, J = 5.8 Hz, 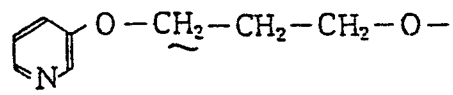 )

4.2 2 ( 2 H, t, J = 5.8 Hz, 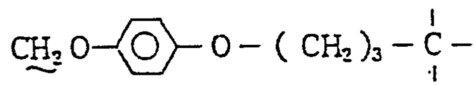 )

6.8 4 ( 4 H, s, 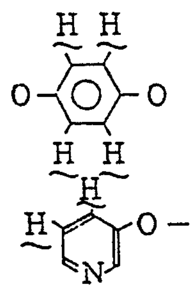 )

7.2 ( 2 H, m, )

8.2 3 ( 1 H, J = 1.8 Hz, 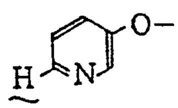 )

8.3 4 ( 1 H, s, 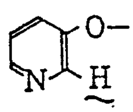 )

0210753

Preparation of Compound J:

Into 5 ml of dry dimethylformamide containing 150 mg of sodium hydride (60% suspension in mineral oil) was added 292 mg of 2-hydroxypyridine. After stirring the mixture at room temperature for 15 minutes, 1.2 g of ethyl 6-[p-(5-bromopentyloxy)phenoxy]-2,2-dimethylhexanoate was added thereto. The mixture was stirred at room temperature for 12 hours, and thereafter the solvent was removed from the reaction solution under reduced pressure. The remaining oily substance was dissolved in chloroform washed with water, and then dried over anhydrous magnesium sulfate. After drying, the solvent was distilled off under reduced pressure. The remaining oily substance was subjected to silica gel column chromatography and the product was eluted using as an eluent chloroform-methanol (99:1). The solvent was distilled off from the eluate under reduced pressure to give the desired ethyl 6-[p-[5-(2-pyridyloxy)pentyloxy)phenoxy]-2,2-dimethylhexanoate as an oily substance (495 mg).

NMR spectra ($CDCl_3$)

δ (ppm)

| | |
|---|---|
| 1.16 (6H, s) | 6.10 (1H, dd) |
| 1.22 (3H, t) | 6.52 (1H, dd) |
| 3.6 - 4.3 (6H, m) | 6.76 (4H, s) |
| 4.10 (2H, q) | |

Mass spectrum

$M^+$ m/z 443

0210753

CLAIMS:

1. The use of at least one compound of the following formula or a salt thereof for the preparation of a medicament for the prevention or treatment of tumors (e.g. mammary or liver or skin tumors) or tumor metastasis in humans or other mammals

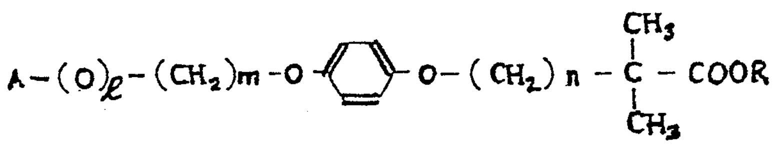

wherein A represents an imidazolyl group or a pyridyl group,

$\ell$ represents 0 or 1,

m and n represent the same or different integers of 1 to 6,

R represents a hydrogen atom or a lower alkyl group.

2. The use for the preparation of a medicament for the prevention or treatment of tumors (e.g. mammary or liver or skin tumors) or tumor metastasis in humans or other mammals of at least one compound selected from ω-[p-(imidazolylalkoxy)phenoxy]-2,2-dimethylalkanoic acid and esters and salts thereof, ethyl 6-[p-[5-(1-imidazolyl)pentyloxy]phenoxy]-2,2-dimethylhexanoate, ethyl 7-[p-[5-(1-imidazolyl)pentyloxy]phenoxy]-2,2-dimethyl-heptanoate hydrochloride, ω-[p-(pyridyloxyalkoxy)phenoxy]-2,2-dimethylalkanoic acid, and esters and salts thereof, and methyl 5-[p-[3-(3-pyridyloxy)propoxy]phenoxy]-2,2-dimethylpentanoate.

0210753

3. Novel compounds of the following formula and salts thereof

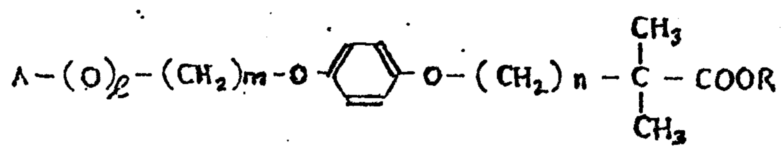

wherein A represents an imidazolyl group or a pyridyl group,

ℓ represents 0 or 1,

m and n represent the same or different integers of 1 to 6; and,

R represents a hydrogen atom or a lower alkyl group.

4. A compound according to claim 3 which is compound F, G or J herein.

5. An anti-tumor medicament comprising at least one compound according to claim 3 or 4.

6. A method of preparing a compound according to claim 4 or 5 which comprises reacting compound

$$A — (O)\ell — M$$

with compound of formula

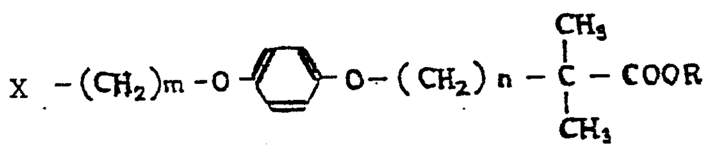

wherein A, $\ell$ and R are as hereinbefore defined, and M is hydrogen or alkali metal and X is a halo- group.